# EUROPÄISCHE PATENTSCHRIFT

(11) **EP 1 517 646 B1**
(45) Veröffentlichungstag und Bekanntmachung des Hinweises auf die Patenterteilung: **10.03.2010**
(21) Anmeldenummer: 03760637.3
(22) Anmeldetag: 17.06.2003
(51) Int. Cl.: A61B 18/14

(54) **ELEKTRODENNADEL**
ELECTRODE NEEDLE
AIGUILLE A ELECTRODE

(30) Priorität: 19.06.2002 DE 10228085
(43) Veröffentlichungstag der Anmeldung: 30.03.2005
(73) Patentinhaber: Celon AG Medical Instruments, 14513 Teltow (DE)
(72) Erfinder: DESINGER, Kai, 12157 Berlin (DE); ROGGAN, André, 12163 Berlin (DE); FAY, Markus, 12249 Berlin (DE); ROTHE, Rainer, 33165 Lichtenau (DE)
(74) Vertreter: von Oppen, Joachim F.M.
(86) Internationale Anmeldenummer: PCT/EP2003/006393
(87) Internationale Veröffentlichungsnummer: WO 2004/000149

(56) Entgegenhaltungen:
- EP-A- 1 059 067
- WO-A-97/13463
- WO-A-99/55244
- US-A1- 2001 039 416
- US-B1- 6 241 725

## Beschreibung

Die Erfindung betrifft eine Elektrodennadel mit einem Schaft und mindestens einer am Schaft ausgebildeten Elektrode.

Eine in der Medizin bekannte Methode zum Behandeln von pathologisch verändertem Körpergewebe ist das elektrochirurgische, insbesondere das elektrothermische Veröden des betroffenen Gewebes. Von besonderem Interesse ist diese Methode für die Therapie von Organtumoren, z. B. Lebertumoren. Zur Verödung werden eine oder mehrere Elektroden im zu verödenden Gewebe, d. h. dem Tumorgewebe, oder in dessen unmittelbarer Nähe platziert und ein Wechselstrom zwischen den Elektroden oder einer Elektrode und einer außen am Körper fixierten, sogenannten Neutralelektrode, fließen gelassen. Fließt der Strom zwischen der Elektrode und der Neutralelektrode (ggf. auch zwischen mehreren Elektroden und einer oder mehreren Neutralelektroden), so spricht man von einer monopolaren Elektrodenanordnung. Fließt der Strom dagegen zwischen den im Gewebe befindlichen Elektroden selbst (in diesem Fall müssen mindestens zwei Elektroden in das Gewebe eingebracht werden), so spricht man von einer bipolaren Anordnung. Die zum Platzieren im Gewebe vorgesehene Elektrode ist in der Regel auf einer Elektrodennadel angeordnet.

Zum Veröden des pathologisch veränderten Gewebes wird mittels Hochfrequenzgenerator ein Stromfluss zwischen den mit dem Körpergewebe in elektrisch leitendem Kontakt stehenden, sog. aktiven Elektroden und beispielsweise einer Neutralelektrode induziert. Der elektrische Widerstand des Körpergewebes führt dabei dazu, dass der Wechselstrom in Wärme umgewandelt wird. Bei Temperaturen zwischen 50°C und 100°C kommt es zu einer massiven Denaturierung der körpereigenen Proteine und in der Folge zum Absterben der betroffenen Gewebeareale. Aufgrund der hohen Stromdichte im Bereich der aktiven Elektroden erfolgt die Erwärmung des Gewebes vorwiegend dort, wo die aktiven Elektroden mit dem Körpergewebe in elektrisch leitfähigem Kontakt stehen.

Im Interesse einer wirkungsvollen Behandlung ist es vorteilhaft, den Fortschritt der Behandlung möglichst zeitnah zu überprüfen. Zu diesem Zweck gehen Mediziner dazu über, das Veröden von Tumorgewebe durch Applikation von Hochfrequenzstrom mittels Kernspintomographie zu überwachen. In einer kernspintomographischen Aufnahme sind dabei nicht nur die Unterschiede zwischen gesundem Gewebe und Tumorgewebe zu erkennen, sondern auch zwischen verödetem und nicht verödetem Gewebe.

Für ein wirkungsvolles Veröden des Tumorgewebes ist aber auch das genaue Platzieren der an einer Elektrodennadel angeordneten aktiven Elektroden im zu verödenden Gewebe oder in dessen Nähe wichtig.

Aus US 6,241,725 B1 ist in diesem Zusammenhang eine Elektrodennadel bekannt, welche zur Überwachung Ihrer Position im Körper eines Patienten über eine aktive Elektrode aus einem MRT-kompatiblen Material verfügt. Die Lokalisation der Elektroden im Körper eines Patienten kann so mit Hilfe von MRT-Aufnahmen während einer Behandlung überprüft werden.

Auch die Aufgabe der Erfindung besteht darin, eine Elektrodennadel zur Verfügung zu stellen, die ein sicheres und genaues Platzieren der aktiven Elektroden im Körper ermöglicht.

Diese Aufgabe wird durch eine Elektrodennadel nach Anspruch 1 gelöst. Die abhängigen Ansprüche enthalten weitere vorteilhafte Ausgestaltungen der erfinderischen Elektrodennadel.

Die erfindungsgemäße Elektrodennadel weist einen Schaft und mindestens eine aktive Elektrode auf, wobei der Schaft ein der aktiven Elektrode räumlich zugeordnetes kernspinaktives Markerelement umfasst und dadurch gekennzeichnet ist, dass das kernspinaktive Markerelement entweder als Beschichtung ausgebildet ist, welche auf der Innenseite eines ein Lumen umgebenden Mantels des Schaftes aufgebracht ist, oder, wenn die aktive Elektrode einen axialen Abschnitt des Schaftes umschließt, entweder als Beschichtung oder als Hülse zwischen dem Schaft und der aktiven Elektrode angeordnet ist. Das kernspinaktive Markerelement setzt sich aus einem Material zusammen, dessen magnetische Eigenschaften sich sowohl von denen des Schaft- und Elektrodenmaterials als auch von denen des Körpergewebes, das sich im wesentlichen aus Wasser zusammensetzt, unterscheidet. Dies kann erfindungsgemäß dadurch erreicht werden, dass Materialien mit paramagnetischen (z.B. Bronze, Aluminium, Kupfer, Messing) oder ferromagnetischen Eigenschaften (z.B. Eisen, Nickel, Stahl) oder dessen Legierungen zur Anwendung kommen.

Der Erfindung liegt der folgende Gedanke zu Grunde:

Konventionelle Elektrodennadeln sind als mit dem Körpergewebe in Kontakt zu bringende Behandlungsvorrichtungen körperverträglich auszugestalten. Der Schaft besteht daher aus körperverträglichen Materialien, bspw. aus körperverträglichen Kunststoffen oder aus körperverträglichen Metallen, die ggf. mit körperverträglichen Kunststoffen überzogen sind. Die aktiven Elektroden sind aus Metall hergestellt und werden entweder von einem Teil des Schaftes gebildet oder sind in diesen integriert. Ein wegen seiner guten Körperverträglichkeit einerseits und einer guten Kernspinkompatibilität andererseits häufig für die aktiven Elektroden oder für Schäfte verwendetes Metall ist Titan oder dessen Legierungen. Dieser Aufbau gewährleistet durch geeignete Artefakte eine gute Bildgebung und Darstellung der gesamten Elektrodennadel.

Von entscheidender Bedeutung für einen guten Behandlungserfolg ist jedoch die optimale Positionierung des aktiven Bereiches der Elektrodennadel, d.h. dem Bereich, der in elektrisch leitendem Kontakt zum umgebenden Körpergewebe steht und in dessen Umgebung durch die hohe Stromdichte die therapeutische, d.h. koagulative Wirkung einsetzt. Dieser Bereich kann bei Elektrodennadeln nach dem Stand der Technik in der magnetresonanz-tomographischen Aufnahme nicht vom restlichen Nadelteil differenziert werden, so dass die Position der Nadel relativ zum pathologischen Gewebe (z.B. Tumor) nur schwer zu bestimmen ist.

Sind die aktiven Elektroden der Elektrodennadel dagegen mit einem kernspinaktiven Markerelement markiert, so hinterlässt das kernspinaktive Markerelement in kernspintomographischen Aufnahmen Artefakte, welche die Lage der aktiven Elektroden sichtbar machen. Da sich in der Aufnahme das zu verödende Gewebe, bspw. ein Tumorgewebe, vom gesunden Gewebe abhebt, ist mit Hilfe der erfindungsgemäßen Elektrodennadel das Überwachen des für die Behandlung vorzunehmenden Platzierens der aktiven Elektroden möglich.

Um nicht nur die Position der aktiven Elektrode der Elektrodennadel in der kernspintomographischen Aufnahme sichtbar zu machen, sondern auch ihre Ausdehnung, erstreckt sich in einer Ausgestaltung der Elektrodennadel das kernspinaktive Markerelement über die gesamte axiale Länge der aktiven Elektrode. Alternativ kann sich das kernspinaktive Markerlement über die gesamte axiale Länge des Schaftes der Elektrodennadel mit Ausnahme der axialen Länge der aktiven Elektrode erstrecken, so dass man in der kernspintomographischen Aufnahme ein Artefakt erhält, in dem der Bereich der aktiven Elektrode ausgespart ist. Man erhält so quasi ein "Negativbild" der aktiven Elektroden.

In einer Ausführungsform der Elektrodennadel ist das kernspinaktive Markerelement als Draht ausgestaltet. Drahtförmige kernspinaktive Markerelemente sind kostengünstig zu produzieren und leicht zu handhaben. Sie können aus einem einfachen ferromagnetisches Material enthaltenden Draht bestehen.

Die Elektrodennadel weist in einer Ausgestaltung der Ausführungsform einen Schaft mit einem Lumen auf. Der Draht ist im Inneren des Lumens, das nicht in Kontakt mit dem Körpergewebe steht, angeordnet. In dieser Ausgestaltung ist es nicht nötig, als kernspinaktives Material ein gut körperverträgliches Material auszuwählen, was die Anzahl der zur Verwendung für die erfindungsgemäße Elektrodennadel geeigneten Materialien erhöht. Der Draht kann beispielsweise an der Innenseite des das Lumen umgebenden Mantels des Schaftes befestigt sein. Eine derartig ausgestaltete Elektrodennadel ist einfach und kostengünstig herstellbar.

In einer alternativen Ausführungsform der erfindungsgemäßen Elektrodennadel ist das kernspinaktive Markerelement als Beschichtung vorhanden. Die Beschichtung kann beispielsweise ferromagnetisches Material enthalten. Die Ausführung als Beschichtung ermöglicht es, in einfacher Weise die gesamte Fläche der aktiven Elektrode zu markieren. Zudem kann die Beschichtung sehr dünn gehalten werden, so dass der Platzbedarf des kernspinaktiven Markerelementes gering ist. Die Beschichtung ist daher insbesondere für sehr dünne Elektrodennadeln geeignet.

Die Elektrodennadel weist in einer Ausgestaltung dieser Ausführungsform, einen Schaft mit einem ein Lumen umgebenden Mantel auf, wobei die Beschichtung an der Innenfläche des Mantels angebracht ist. Da die Beschichtung dünn ausgeführt werden kann, beansprucht sie keinen Platz, der für andere Komponenten der Elektrodennadel, wie bspw. die elektrische Zuleitung zur aktiven Elektrode und/oder eine Kühlmittelzuleitung zum Kühlen der aktiven Elektrode, vorgesehen ist.

In einer alternativen Ausgestaltung der Ausführungsform umschließt die aktive Elektrode einen axialen Abschnitt des Schaftes. Die Beschichtung ist in dieser Ausgestaltung zwischen dem Schaft und der aktiven Elektrode entweder am Schaft oder an der Elektrode angebracht.

Statt als Beschichtung oder Draht kann das kernspinaktive Markerelement in einer weiteren Ausführungsform der erfindungsgemäßen Elektrodennadel als Hülse ausgestaltet sein. Eine Hülse ist leicht herzustellen und zu handhaben.

In einer Ausgestaltung dieser Ausführungsform umschließt die aktive Elektrode einen axialen Abschnitt des Schaftes. Die Hülse ist hierbei zwischen dem Schaft und der aktiven Elektrode angeordnet.

In einer weiteren Ausführungsform ist das kernspinaktive Markerelement als Drahtspule und insbesondere als Helixfeder ausgestaltet. Eine Drahtspule kann, insbesondere wenn sie als Feder ausgestaltet ist, in einfacher Weise mittels Klemmsitz im Inneren der Nadel befestigt werden. Eine Drahtspule hat aufgrund ihrer Induktivität auch dann kernspinaktive Wirkung, wenn sie kein ferromagnetisches Material enthält.

Die Drahtspule kann zudem in einer vorteilhaften Weiterbildung der Ausführungsform auf die Frequenz des Kernspintomographen abgestimmt sein. Das Abstimmen ermöglicht es, die Intensität eines von der Drahtspule in der kernspintomographischen Abbildung hinterlassenen Artefaktes an bestehende Anforderungen anzupassen.

Weitere vorteilhafte Eigenschaften, Merkmale und Ausgestaltungen der erfindungsgemäßen Elektrodennadel ergeben sich unter Bezugnahme auf die beiliegenden Zeichnungen aus der nachfolgenden detaillierten Beschreibung verschiedener Ausführungsbeispiele.
- Figur 1: zeigt eine Elektrodennadel in perspektivischer Darstellung.
- Figur 2: zeigt das distale Ende der in Figur 1 dargestellten Elektrodennadel in einer vergrößerten Darstellung.
- Figur 3: zeigt ein erstes Ausführungsbeispiel für die erfindungsgemäße Elektrodennadel in einem Schnitt entlang ihrer Längsachse.
- Figur 4: zeigt eine alternative Ausgestaltung des ersten Ausführungsbeispiels in einem Schnitt entlang der Längsachse.
- Figur 5: zeigt ein zweites Ausführungsbeispiel für die erfindungsgemäße Elektrodennadel in einem Schnitt entlang der Längsachse.
- Figur 6: zeigt eine alternative Ausgestaltung des zweiten Ausführungsbeispiels in einem Schnitt entlang der Längsachse.
- Figur 7: zeigt ein drittes Ausführungsbeispiel für die erfindungsgemäße Elektrodennadel in einem Schnitt entlang der Längsachse.
- Figur 8: zeigt ein viertes Ausführungsbeispiel für die erfindungsgemäße Elektrodennadel in einem Schnitt entlang der Längsachse.
- Figur 9: zeigt eine alternative Ausgestaltung des vierten Ausführungsbeispiels in einem Schnitt entlang der Längsachse.
- Figur 10: zeigt eine weitere alternative Ausgestaltung in einem Schnitt entlang der Längsachse.

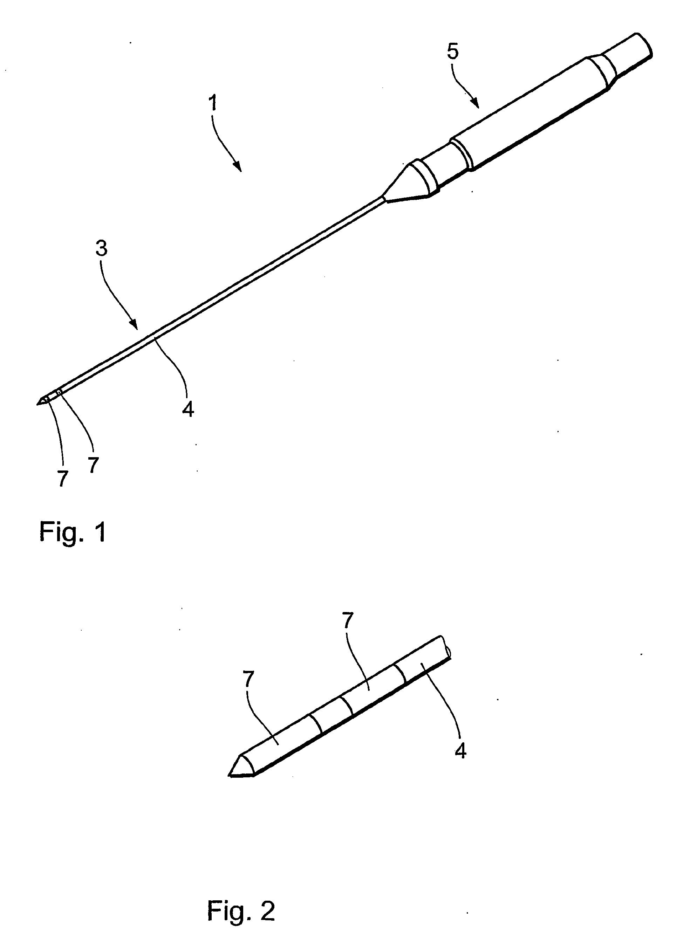

In Figur 1 ist in perspektivischer Darstellung eine Elektrodennadel 1 zu sehen. Die Elektrodennadel 1 umfasst einen Schaftabschnitt 3 mit einem Schaft 4, der an seinem distalen Ende zwei aktive Elektroden 7 aufweist. Zudem weist die Elektrodennadel 1 einen Griffabschnitt 5 zum Handhaben der Nadel auf.

Eine vergrößerte Darstellung des distalen Endes des Schaftes 4 mit den beiden aktiven Elektroden 7 ist in Figur 2 dargestellt. Zwar sind in den Figuren 1 und 2 am distalen Ende des Schaftes 4 jeweils zwei aktive Elektroden dargestellt, jedoch kann die Elektrodennadel 1 je nach Anwendungszweck (monopolare, bipolare oder multipolare Behandlung) eine beliebige Anzahl aktiver Elektroden 7 umfassen. Mindestens ist jedoch eine aktive Elektrode 7 vorhanden.

Als Materialien für den Schaft kommen körperverträgliche Materialien, insbesondere Kunststoffe oder Metalle, in Frage. Der Schaft kann, wenn er aus Metall hergestellt ist, in Abschnitten, in denen er isolierend sein soll, mit einem elektrisch isolierenden Überzug, beispielsweise einem Lack- oder Kunststoffüberzug, versehen sein. Für die aktiven Elektroden 7 und metallische Schäfte wird üblicherweise aufgrund seiner guten Körperverträglichkeit Titan oder eine Titanlegierung verwendet. Aufgrund ihrer paramagnetischen Eigenschaften sind diese Materialien kernspinkompatibel. Auch andere, paramagnetische und körperverträgliche Metalle kommen grundsätzlich in Frage.

Der Schaft 4 der Elektrodennadel 1 ist im vorliegenden Ausführungsbeispiel hohl ausgebildet, d. h. er umfasst einen Mantel 10, der ein Lumen 8 umschließt. Das Lumen 8 dient dabei üblicherweise zur Aufnahme elektrischer Leitungen zum Anschließen eines nicht dargestellten Hochfrequenzgenerators an die aktiven Elektroden 7 sowie gegebenenfalls von Kühlmittelzuführleitungen zum Kühlen der aktiven Elektroden 7 im Betrieb.

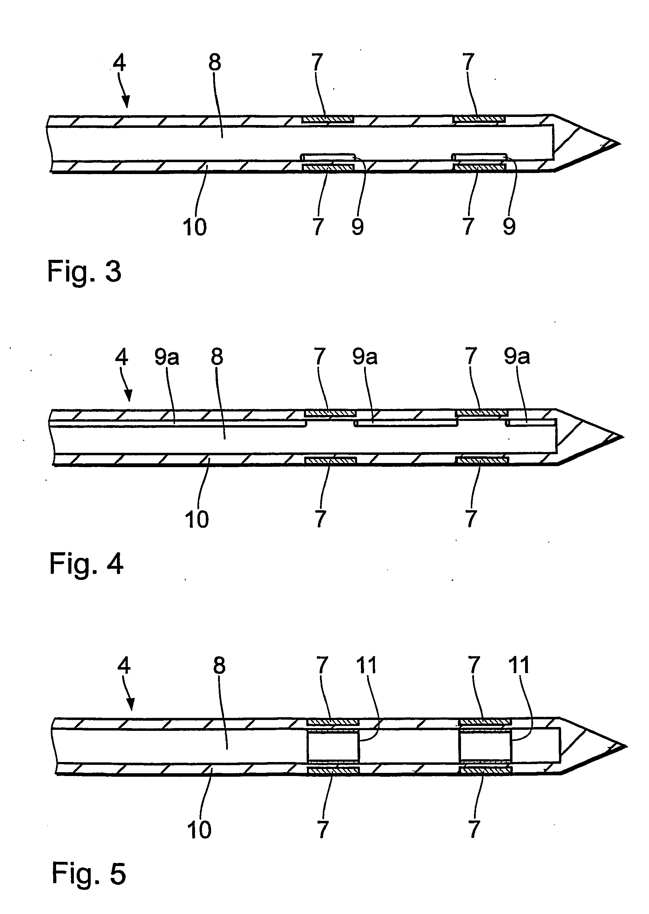

Ein erstes Ausführungsbeispiel für die erfindungsgemäße Elektrodennadel 1 ist als Schnitt entlang der Längsachse des Schaftes 4 in Figur 3 dargestellt. Im Schnitt sind der Schaft 4, der Mantel 10 sowie das Lumen 8 der Nadel zu erkennen. An der Außenfläche des Mantels 10 sind die aktiven Elektroden 7 angeordnet, die den Mantel 10 ringförmig umschließen und sich über eine bestimmte axiale Länge des Mantels erstrecken. Die axiale Länge kann, anders als in Figur 3 dargestellt, für jede aktive Elektrode verschieden sein. Der Mantel 10 ist bei der hier abgebildeten, bipolaren Elektrodennadel aus einem isolierenden Material hergestellt, um die beiden aktiven Elektroden 7 gegeneinander zu isolieren, und weist dort, wo die aktiven Elektroden 7 angeordnet sind, eine gegenüber dem Rest des Schaftes 4 geringere Wandstärke auf, damit die Elektroden 7 bündig mit der Außenfläche des Mantels 10 abschließen.

An der Innenwand des Mantels 10 sind dort, wo sich die aktiven Elektroden 7 befinden, Drahtstücke 9 aus ferromagnetischem Material, beispielsweise Stahldraht, angeordnet. Der Draht 9 kann z.B. an die Innenfläche des Mantels 10 geklebt, gelötet oder punktgeschweißt sein. Sein Durchmesser ist vorteilhafterweise so groß, dass er in einer kernspintomographischen Aufnahme gut zu erkennen ist, aber auch klein genug, damit im Lumen 8 genügend Raum für weitere darin anzuordnende Komponenten der Elektrodennadel 1 verbleibt.

Im dargestellten Ausführungsbeispiel ist der Draht 9 zwischen den beiden Elektroden 7 unterbrochen, so dass beide Elektroden separat markiert sind. Alternativ ist es auch möglich, den gesamten aktiven Bereich der Elektrodennadel 4, der von beiden Elektroden 7 und der zwischen ihnen liegenden Isolation gebildet wird, einheitlich mit einem durchgehenden Stück Draht zu markieren. Diese kostengünstige Alternative bringt kaum Nachteile mit sich, da in der Regel nur der von allen Elektroden gemeinsam gebildete aktive Bereich einer Elektrodennadel interessiert.

Der Draht 9 erstreckt sich jeweils über die gesamte axiale Länge einer aktiven Elektrode 7, so dass sein Bild in einer kernspintomographischen Aufnahme nicht nur die Position, sondern auch die Länge der aktiven Elektrode 7 anzeigt.

Figur 4 zeigt eine alternative Ausgestaltung des ersten Ausführungsbeispiels. Vom ersten Ausführungsbeispiel unterscheidet sie sich dadurch, dass sich der kernspinaktive Draht 9a über die gesamte Länge des Schaftes 4 mit Ausnahme derjenigen Bereiche, in denen sich die aktiven Elektroden 7 befinden, erstreckt. In einer kernspintomographischen Aufnahme führt dies dazu, dass die Abschnitte, in denen sich die aktiven Elektroden 7 befinden, von Wiedergaben des Drahtes 9a begrenzt werden. In diesem Sinne stellt das kernspintomographische Bild, welches mittels der in Figur 4 dargestellten Ausgestaltung gewonnen wird, das Negativbild desjenigen Bildes dar, das mit der Ausgestaltung aus Figur 3 gewonnen wird.

Ein zweites Ausführungsbeispiel für die erfindungsgemäße Elektrodennadel ist in Figur 5 dargestellt. Im folgenden wird nur auf die Unterschiede zum ersten Ausführungsbeispiel eingegangen.

Anders als im ersten Ausführungsbeispiel ist das kernspinaktive Markerelement statt als ferromagnetischer Draht als ferromagnetische Beschichtung 11 auf die Innenfläche des Mantels 10 aufgebracht. Die Beschichtung 11 erstreckt sich jeweils auf der axialen Länge einer aktiven Elektrode 7 über den gesamten Innenumfang des Mantels 10. Alternativ kann sich die Beschichtung auch auf den gesamten aktiven Bereich erstrecken, der von beiden Elektroden und der zwischen ihnen liegenden Isolation gebildet wird.

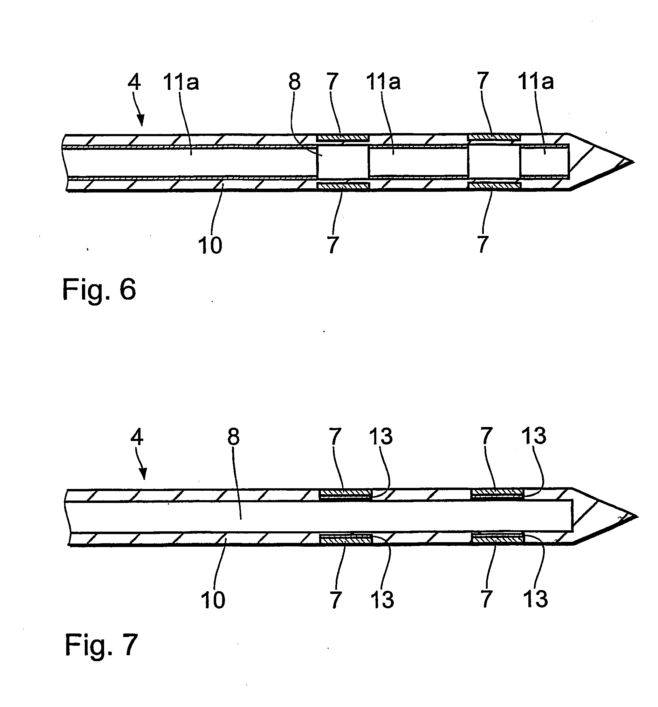

In einer alternativen Ausgestaltung (siehe Figur 6) ist die gesamte Innenfläche des Mantels 10 bis auf diejenigen Bereiche, in denen sich die aktiven Elektroden 7 befinden, beschichtet. Wie im ersten Ausführungsbeispiel erzeugt diese Ausgestaltung in einer kernspintomographischen Aufnahme quasi ein Negativbild der aktiven Elektroden 7.

Ein drittes Ausführungsbeispiel für die erfindungsgemäße Elektrodennadel 1 ist in Figur 7 dargestellt. In dieser Ausführungsform ist das kernspinaktive Material zwischen der Innenfläche der aktiven Elektroden 7 und der Außenfläche des Mantels 10 angeordnet.

In der dargestellten Ausgestaltung liegt das kernspinaktive Markerelement in Form einer Hülse 13, beispielsweise einer Stahlhülse, vor, welche die Außenfläche des Mantels 10 ringförmig umgibt. Vorteilhafterweise weist die Außenfläche des Mantels 10 dort, wo die aktiven Elektroden 7 anzubringen sind, Ringnuten auf, die zur Aufnahme der Hülse 13 geeignet sind. Um die Hülse herum sind dann die aktiven Elektroden 7 angeordnet. Die Tiefe der Nuten ist vorzugsweise so gewählt, dass die Außenflächen der ringförmigen Elektroden 7 bündig mit der Außenfläche des Mantels 10 abschließen. Auch in den übrigen dargestellten Ausführungsbeispielen ist es vorteilhaft, wenn die Außenflächen der aktiven Elektroden 7 bündig mit der Außenfläche des Mantels 10 abschließen.

Zwar ist im dritten Ausführungsbeispiel das kernspinaktive Markerelement als ferromagnetische Hülse ausgestaltet, jedoch können als Alternative auch die aktiven Elektroden 7 als Hülsen ausgestaltet sein, deren innere Umfangsfläche mit einem ferromagnetischen Material beschichtet ist. In einer weiteren Alternative können die aktiven Elektroden 7 ebenfalls als Hülsen ausgestaltet sein, wobei die Beschichtung jedoch an den Bodenflächen der Ringnuten vorhanden ist.

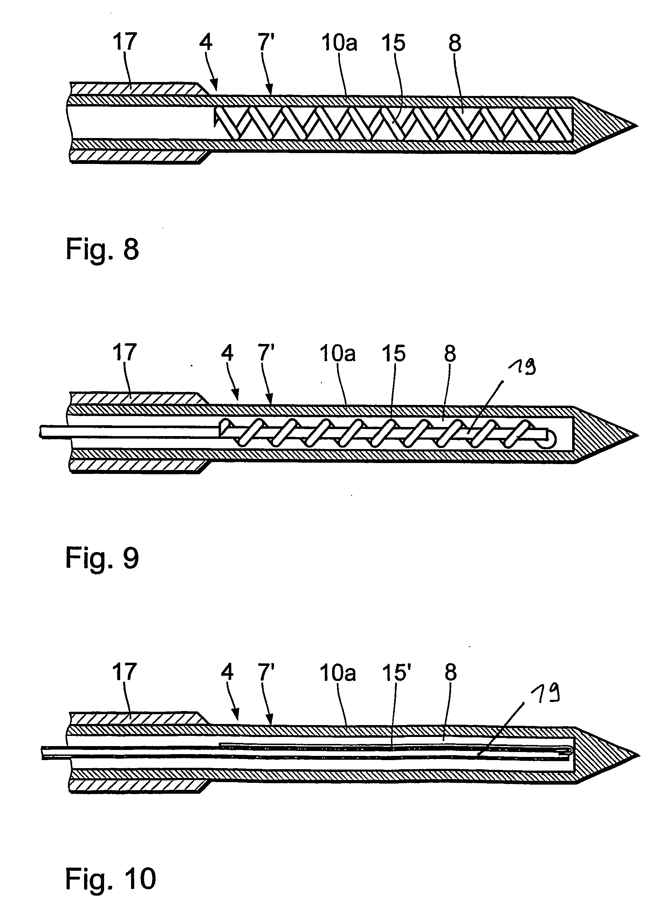

Ein viertes Ausführungsbeispiel für die erfindungsgemäße Elektrodennadel 1 ist in Figur 8 dargestellt. Die Elektrodennadel dieses Ausführungsbeispiels umfasst einen metallischen Schaft 4, bspw. aus Titan, mit einem Lumen 8 und einem das Lumen 8 umgebenden Mantel 10a. Sie umfasst außerdem eine die Außenfläche des Schaftes 4 umschließende isolierende Hülle 17. Das distale Ende des Schaftes 4 ragt aus der isolierenden Hülle 17 heraus und bildet die einzige aktive Elektrode 7' der dargestellten Elektrodennadel 1.

Im inneren des Lumens 8 ist eine Drahtspule 15 angeordnet, die sich vom distalen Ende des Schaftes bis zum Beginn der isolierenden Hülle 17 erstreckt. Sie kann z.B. durch Verlöten, Verschweißen, Verkleben oder Verklemmen an der Innenseite des Schaftes 4 befestigt sein.

Eine alternative Ausgestaltung dieses Ausführungsbeispiels ist in Figur 9 gezeigt. Im Lumen 8 des Schaftes 4 ist eine Zuleitung 19 zum Zuführen eines Kühlmittels angeordnet. Die Drahtspule 15 aus einem kemspinaktiven Material ist vom distalen Ende der Zuleitung 19, wo sie in die Öffnung der Zuleitung eingehängt ist, bis zum Beginn der isolierenden Hülle 17 um die Zuleitung gewickelt. Der in die Öffnung der Zuleitung 19 eingehängte Abschnitt der Drahtspule 15 ist so lang, dass er selbst dann, wenn die Drahtspule 15 verrutscht und an das distale Ende des Lumens 8 anstößt, noch teilweise in die Öffnung der Zuleitung 19 hineinragt und so ein völliges Lösen der Spule 15 von der Zuleitung 19 verhindert.

Die Drahtspule 15 kann in beiden Ausgestaltungen, als Feder ausgestaltet, auch mit Klemmsitz im Lumen 8 oder um die Zuleitung 19 herum befestigt sein.

Statt einer Drahtspule kann alternativ auch ein gerades Stück Draht 15' aus ferromagnetischem Material in die Zuleitung eingehängt sein. Diese Variante ist in Figur 10 abgebildet.

## Patentansprüche

1. Elektrodennadel mit einem Schaft (4) und mindestens einer am Schaft (4) ausgebildeten aktiven Elektrode (7), wobei der Schaft (4) ein der aktiven Elektrode (7) räumlich zugeordnetes kernspinaktives Markerelement (11; 11a; 13,) umfasst, **dadurch gekennzeichnet, dass** das kernspinaktive Markerelement entweder als Beschichtung (11, 11a) ausgebildet ist, welche auf der Innenseite eines ein Lumen (8) umgebenden Mantels (10) des Schaftes (4) aufgebracht ist, oder, wenn die aktive Elektrode (7) einen axialen Abschnitt des Schaftes (4) umschließt, entweder als Beschichtung oder als Hülse (13) zwischen dem Schaft (4) und der aktiven Elektrode (7) angeordnet ist.

2. Elektrodennadel nach Anspruch 1, **dadurch gekennzeichnet, dass** sich das kernspinaktive Markerelement (11; 13,) über die gesamte axiale Länge der aktiven Elektrode (7) erstreckt.

3. Elektrodennadel nach Anspruch 1, **dadurch gekennzeichnet, dass** sich das kernspinaktive Markerelement (11; 13,) über die gesamte axiale Länge mehrerer aktiver Elektroden (7) und der zwischen ihnen liegenden Zwischenräume erstreckt.

4. Elektrodennadel nach Anspruch 1, **dadurch gekennzeichnet, dass** sich das kernspinaktive Markerelement (11a) über die gesamte axiale Länge des Schaftes (4) mit Ausnahme der axialen Länge der aktiven Elektrode (7) erstreckt.

## Claims

1. Electrode needle comprising a shaft (4) and at least one active electrode (7) provided on the shaft (4), wherein the shaft (4) includes a nuclear magnetic resonance-active marker element (11; 11 a; 13) which is spatially associated with the active electrode (7), **characterised in that** the nuclear magnetic resonance-active marker element is either in the form of a coating (11, 11 a) which is applied onto the inside of a casing (10) of the shaft (4) surrounding a lumen (8), or, if the active electrode (7) encloses an axial section of the shaft (4), either as a coating or as a sleeve (13) between the shaft (4) and the active electrode (7).

2. Electrode needle according to claim 1, **characterised in that** the nuclear magnetic resonance-active marker element (11; 13) extends over the entire axial length of the active electrode (7).

3. Electrode needle according to claim 1, **characterised in that** the nuclear magnetic resonance-active marker element (11; 13) extends over the entire axial length of a plurality of active electrodes (7) and the intermediate spaces between them.

4. Electrode needle according to claim 1, **characterised in that** the nuclear magnetic resonance-active marker element (11 a) extends over the entire axial length of the shaft (4) with the exception of the axial length of the active electrode (7).

## Revendications

1. Aiguille d'électrode comprenant une tige (4) et au moins une électrode active (7) conçue sur la tige (4), la tige (4) comportant un élément de marquage (11 ; 11a ; 13) fonctionnant par résonance magnétique nucléaire associé dans l'espace à l'électrode active (7), **caractérisée en ce que** l'élément de marquage fonctionnant par résonance magnétique nucléaire est soit conçu comme un revêtement (11, 11 a), lequel est appliqué sur la face intérieure d'une enveloppe (10) de la tige (4) entourant une lumière (8), soit, lorsque l'électrode active (7) entoure une partie axiale de la tige (4), disposé comme revêtement ou comme gaine (13) entre la tige (4) et l'électrode active (7).

2. Aiguille d'électrode selon la revendication 1, **caractérisée en ce que** l'élément de marquage (11 ; 13) fonctionnant par résonance magnétique nucléaire s'étendant au-dessus de toute la longueur axiale de l'électrode active (7).

3. Aiguille d'électrode selon la revendication 1, **caractérisée en ce que** l'élément de marquage (11 ; 13) fonctionnant par résonance magnétique nucléaire s'étend au-dessus de toute la longueur axiale de plusieurs électrodes actives (7) et des espaces intermédiaires situés entre celles-ci.

4. Aiguille d'électrode selon la revendication 1, **caractérisée en ce que** l'élément de marquage (11a) fonctionnant par résonance magnétique nucléaire s'étend au-dessus de toute la longueur axiale de la tige (4) à l'exception de la longueur axiale de l'électrode active (7).
